# EUROPÄISCHE PATENTSCHRIFT

(11) **EP 0 591 182 B1**
(45) Veröffentlichungstag und Bekanntmachung des Hinweises auf die Patenterteilung: **13.05.1998**
(21) Anmeldenummer: 91912024.6
(22) Anmeldetag: 25.06.1991
(51) Int. Cl.: B05B 11/02, G01F 11/02

(54) **AUSTRAGVORRICHTUNG FÜR MEDIEN**
DISPENSING DEVICE FOR MEDIA
APPLICATEURS DE MILIEUX

(30) Priorität: 04.07.1990 DE 4021263
(43) Veröffentlichungstag der Anmeldung: 13.04.1994
(60) Teilanmeldung: 97118764.6
(73) Patentinhaber: Ing. Erich Pfeiffer GmbH, 78315 Radolfzell (DE)
(72) Erfinder: GRAF, Lothar, D-7703 Rielasingen-Worblingen (DE); FUCHS, Karl-Heinz, D-7760 Radolfzell (DE)
(74) Vertreter: Patentanwälte Ruff, Beier, Schöndorf und Mütschele
(86) Internationale Anmeldenummer: EP9101181
(87) Internationale Veröffentlichungsnummer: WO9200812

(56) Entgegenhaltungen:
- EP-A- 0 311 863
- EP-A- 0 334 349
- FR-A- 2 625 981
- US-A- 4 627 432

## Beschreibung

Die Erfindung betrifft eine Austragvorrichtung nach dem Oberbegriff des Patentanspruches 1. Sie ist für fließfähige Medien geeignet, die pulver-, gasförmig und/oder pastös, insbesondere jedoch flüssig sein können und meist der kosmetischen oder pharmazeutischen Anwendung dienen. Zweckmäßig ist die Austragvorrichtung zum Austrag durch eine manuelle Hub- bzw. Arbeitsbewegung ausgebildet.

Die Anwendung derartiger Medien kann in einzelnen, aufeinanderfolgenden, stofflich oder im Volumen gleichen bzw. verschiedenen Chargen zweckmäßig sein. Hierzu können zwar Austragvorrichtungen mit Zählwerken für die ausgebrachten Chargenmengen vorteilhaft sein, jedoch ergibt sich hier eine verhältnismäßig komplizierte und raumaufwendige Ausbildung. Auch können mit üblichen Pumpendosierern aufeinanderfolgend gleich oder unterschiedlich große Chargen ausgebracht werden, wenn eine einstellbare Dosiereinrichtung vorgesehen ist. In diesem Fall kann jedoch die Anzahl der bereits ausgebrachten Chargen nicht festgestellt werden.

Die FR-A-2 625 981 sowie die EP-A-0 311 863 zeigen Austragvorrichtungen, bei welchen der einteilige Medien-Behälter einen von zwei gegeneinander bewegbaren Körpern bildet, von denen der andere als Austragkopf mit dem Medienauslaß versehen ist. Der Behälterkörper liegt in Ausgangsstellung im wesentlichen frei, wobei er nur auf kurzer Länge geführt sowie für seine Montage am Austragkopf für sich genau auszurichten ist.

Der Erfindung liegt auch die Aufgabe zugrunde, Nachteile bekannter Ausbildungen zu vermeiden und insbesondere eine Austragvorrichtung zu schaffen, welche sich einfach herstellen, montieren und handhaben läßt.

Erfindungsgemäß sind die Merkmale des Patentanspruches 1 vorgesehen. Ferner ist auch vorgesehen, Austragchargen, ggf. in genau vorbestimmter Reihenfolge, aufeinanderfolgend auf einfache Weise ausbringen zu können. Für diesen Zweck kann die Austragvorrichtung von der Ausgangsstellung für einen Betätigungshub auf die Ausgangsstellung für den nächsten Betätigungshub unabhängig davon umschaltbar sein, wie groß das Austragvolumen durch den jeweiligen Betätigungshub ist. Im Falle eines nicht gekrümmten, sondern im wesentlichen linearen Betätigungshubes kann die Pumpe schrittweise in ihrer Hubrichtung von Ausgangsstellung zur Ausgangsstellung z.B. so überführt werden, daß die Hubendstellung eines Betätigungshubes die Ausgangsstellung für den nächsten Betätigungshub ist. Die Umschaltung kann aber auch stattdessen oder zusätzlich hierzu quer zur Hubrichtung erfolgen, so daß aufeinanderfolgend aus unterschiedlichen Pumpenkammern ausgetragen wird.

Die erfindungsgemäße Ausbildung eignet sich insbesondere zum Austrag einer Vielzahl, z.B. von sieben, vierzehn oder mehr Austragchargen, wie es erforderlich ist, wenn z.B. ein Wirkstoff jeden Tag einmal über eine, zwei oder mehr Wochen angewandt werden soll. Trotz dieser verhältnismäßig großen Anzahl von Schaltstufen kann die Austragvorrichtung sehr kompakt ausgebildet werden. Beim Erfindungsgegenstand kann am Ende jedes vorangehenden Austragzyklus' eine selbsttätig einrückende bzw. wirksam werdende Sperrung des folgenden Austragzyklus' durch die Folgeschaltung vorgesehen sein, die zur Freigabe dieses folgenden Austragzyklus' zuerst manuell zu betätigen ist, wonach erst die Sperrung aufgehoben ist und der folgende Austragzyklus durchgeführt werden kann. Dadurch erst ist eine bestimmte Reihenfolge von Austragchargen festzulegen, weil nämlich die Folgeschaltung die Aufeinanderfolge zweier oder mehrerer Austragchargen so festlegt, daß zwischen diesen eine Sperrung der Austragmöglichkeit liegt, die erst durch eine gesonderte Freigabebetätigung wieder aufgehoben werden kann.

Zweckmäßig weist die Austragvorrichtung als Austrageinheit mindestens eine Schubkolbenpumpe bzw. einen einzigen oder mehrere im wesentlichen formstabile Pumpenzylinder bzw. Medien-Speicher und nicht zerstörbare Speicherkapseln für das Medium auf, so daß der Pumpenzylinder z.B. aus Glas gefertigt werden kann und sehr hohe hygienische Anforderungen erfüllt. Dies wird noch weiter verbessert, wenn die befüllte Austragvorrichtung das Medium nur in mindestens einem solchen Behälter enthält, der gleichzeitig den Pumpenzylinder für die Führung eines Pumpkolbens bildet und diesem in Ausgangsstellung im Abstand gegenüberliegend am Boden dicht geschlossen ist, nämlich keine Einlaß- oder Ansaugöffnung aufweist.

Damit die Austragvorrichtung stets nur in der richtigen Richtung weitergeschaltet werden kann, ist ein gegen die entgegengesetzte Richtung arretierendes Gesperre vorgesehen, das nach Art eines Freilaufes ausgebildet sein kann.

Bei der beschriebenen oder einer anderen Ausbildung der Austragvorrichtung kann auch vorteilhaft vorgesehen sein, daß wenigstens ein Teil des Kolbenstößels einer Kolbeneinheit in einer Ausgangsstellung im wesentlichen vollständig von der Kolbeneinheit getrennt ist und erst durch eine Schwenkbewegung und/oder eine Hubbewegung mit dem Pumpkolben gekuppelt werden kann. Befindet sich hierbei der Pumpkolben bereits innerhalb des Pumpenzylinders, so kann er einen dichten Verschluß des in der Pumpenkammer gespeicherten Mediums bilden. Der Verschluß wird beim Ankuppeln des Kolbenstößels selbsttätig, z.B. durch Zerstörung, geöffnet und dabei gleichzeitig die Leitungsverbindung zwischen der Pumpenkammer und einem Auslaßkanal bzw. einer Auslaßöffnung hergestellt. Da das Medium bei einer solchen Ausbildung völlig dicht versiegelt in der Pumpenkammer gespeichert werden kann, können sehr große Lagerzeiten erreicht werden. Außerdem kann der Kupplungsvorgang so gestaltet sein, daß er eine Sicherung gegen unbefugte Benutzung, z.B. durch Kinder, bildet.

Die Austragvorrichtung kann sehr einfach ausgebildet werden. Z.B. weist sie zwei im wesentlichen einteilige, teleskopartig ineinandergreifende Kappen auf, deren voneinander abgekehrte, freiliegende Stirnwände unmittelbar Druck-Handhaben zur Hubbetätigung bilden, während ihre freiliegenden Außenumfänge Greif-Handhaben zur Verdreh-Betätigung bilden, die der Weiterschaltung dient. Über eine dieser Stirnwände steht zweckmäßig ein den Kolbenstößel tragender Austragstutzen vor, während die andere Stirnwand die zugehörige Endbegrenzung der Austragvorrichtung bildet. Die zugehörige Kappe weist im Innern und nach außen vollständig verdeckt mindestens eine Aufnahme zur Steckbefestigung eines Speicher- bzw. Zylinderbehälters auf.

Besonders vorteilhaft ist es, wenn eine Mehrzahl von Speicher- bzw. Zylinderbehältern in einer Reihe oder einem Kreis-Kranz so angeordnet sind, daß mit der genannten Folgeschaltung ein Behälter nach dem anderen, insbesondere durch dieselbe Austragöffnung entleert werden kann. Dadurch wird praktisch für jeden Austrag ein neuer, versiegelter Behälter angebrochen, der z.B. ein Speichervolumen von einer Größenordnung von nur etwa 0,1 ml zu haben braucht. Eine solche Austragvorrichtung kann sehr kleine Abmessungen von z.B. etwa 5 cm Durchmesser und - ohne Austragstutzen - demgegenüber kleinerer Axialerstreckung haben.

Diese und weitere Merkmale von bevorzugten Weiterbildungen der Erfindung gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Ausführungsbeispiele der Erfindung sind in den Zeichnungen dargestellt und werden im folgenden näher erläutert. In den Zeichnungen zeigen:
- Fig. 1: eine erfindungsgemäße Austragvorrichtung in Ansicht und etwa natürlicher Größe,
- Fig. 2: die Austragvorrichtung, vergrößert im Axialschnitt,
- Fig. 3: einen Querschnitt durch die Anordnung gemäß Fig. 2,
- Fig. 4: eine weitere Ausbildung im Axialschnitt,
- Fig. 5: einen Querschnitt durch die Fig. 4,
- Fig. 6: einen Ausschnitt der Fig. 5 in vergrößerter Darstellung und
- Fig. 7: eine Ansicht auf den Innenumfang der Kappe gemäß Fig. 4 in einem abgewickelten Ausschnitt.

Die Austragvorrichtung 1 ist an ihrer Außenseite im wesentlichen von zwei Gehäusekörpern 2, 3 begrenzt, die nach Art einer flachen Dose mit Deckel ineinandergreifen und im Innern vollständig nach außen abgedeckt eine Pumpenanordnung 4 mit einer Vielzahl von Pumpen 6 aufnehmen.

Jeder Pumpe 6 ist ein Zylinder 7 einer Zylinderanordnung 5 zugeordnet, der mit einem Pumpkolben 9 einer Kolbeneinheit 8 eine Montage-Baueinheit bildet. Mit einer Folgeschaltung 10 kann jede Pumpe aus einer Ruhelage in eine Arbeitslage überführt werden, in welcher sie zur Betätigung mit einem Stößel 11 etwa achsgleich ausgerichtet ist und ihre durch einen Behälter 13 gebildete Pumpenkammer 12 über einen Auslaßkanal 15 durch eine Auslaßöffnung 14 unmittelbar ins Freie entleert werden kann.

Zu diesem Zweck weist jede Pumpe 6 in einer achsgleichen Durchgangsöffnung 16 ihres hülsenförmigen Pumpkolbens 9 einen Verschluß 17 in Form beispielsweise einer sehr dünnen Membran auf, die mit ihrem Rand an der Innenfläche der Durchgangsöffnung 16 dicht befestigt ist. Der Pumpkolben 9 liegt in Ausgangslage vollständig innerhalb des Behälters 13 benachbart zu dessen auf voller Innenweite des Zylinders 7 offenem Ende und bildet mit dem Verschluß 17 einen die Pumpenkammer 12 dicht nach außen verschließenden Verschlußstopfen. Der Pumpkolben 9 weist z.B. drei axial hintereinander liegende, einteilig mit der Kolbenhülse ausgebildete Dichtlippen auf, die ausschließlich zu seiner Halterung und Führung an der Zylinderlaufbahn dienen.

Boden und Mantel jedes Behälters 13 sind einteilig ausgebildet, und der Verschluß 17 liegt bei dem dem Boden zugekehrten Ende des Pumpkolbens 9 bzw. der Durchgangsöffnung 16. Alle Pumpen 5 sind annähernd achsparallel zueinander, zum Stößel 11 und zum jeweiligen Gehäusekörper 2 bzw. 3 so angeordnet, daß ihre Enden jeweils etwa in einer gemeinsamen Ebene liegen.

Der im wesentlichen zylindrische und in der Außenweite kleinere Grund- bzw. Gehäusekörper 2 weist zwei annähernd koaxial ineinanderliegende Mäntel 18, 19 auf, die über etwa radiale Stege 22 einteilig miteinander verbunden sind und zwischen jeweils zwei Stegen eine Halterung 23 für eine Pumpe 5 bzw. einen Behälter 13 bilden. Die Achsen 20 der Halterungen 23 liegen in einem Kranz um die gemeinsame Achse 21 des Gehäuses, von der die Mittelachse des Stößels 11 denselben Abstand hat. Am Boden jeder Halterung 23, die eine eng an den Behälter 13 angepaßte, axiale Stecköffnung bildet, ist eine Stütze 24, z.B. mindestens eine Rippe, vorgesehen, an welcher der zugehörige Behälter 13 mit seinem Boden im Abstand vom Boden der Stecköffnung abgestützt ist.

Die offene Stirnfläche des jeweiligen Behälters 13 wird von einer ringwulstförmigen Rast 25 des Halterungsmantels übergriffen, wodurch sich eine spielfreie Schnappbefestigung des Behälters 13 ergibt, der annähernd bis an die offene, innere Stirnseite des Gehäusekörpers 2 reicht. An der äußeren Stirnseite ist der Gehäusekörper 2 mit einer annähernd ebenen Stirnwand 26 verschlossen. In Ausgangslage ragt der Gehäusekörper 2 über den größten Teil seiner Länge aus dem Gehäusekörper 3. Dieser weist ebenfalls zwei koaxial ineinanderliegende Mäntel 27, 28 auf, zwischen denen ein Ringraum 29 für den drehbaren und axial verschiebbaren Eingriff des ringförmigen Halterungsvorsprunges des Gehäusekorpers 2 begrenzt ist.

Die Führung 30 ist im weseltlichen nur durch die inneren Mäntel 18, 27 gebildet, wobei eine Wulst 31 am inneren Ende des Mantels 18 am Innenumfang des Mantels 27 und eine Wulst 32 des inneren Endes des Mantels 27 am Innenumfang des Mantels 18 gleitet. Diese Wulste 31, 32 bilden gleichzeitig Schnappglieder zur einfachen Montageverbindung der Gehäusekörper 2, 3 und die einzige Axialsicherung zur Festlegung der Ausgangsstellung, in welcher sie mit Ringschultern aneinander anliegen. Die Mäntel 27, 28 sind ebenfalls einteilig mit einer im wesentlichen ebenen Stirnwand 33 ausgebildet, die mit der Stirnwand 26 die Endbegrenzungen des Gehäuses bildet und über die nur ein den Stößel 11 versenkt aufnehmender und in seiner Endfläche die Auslaßöffnung 14 aufweisender Stutzen 34 vorsteht.

Ein äußerer Stutzenmantel schließt einteilig an die Stirnwand 33 an und geht bei seinem freien Ende in einen innen liegenden Stößelmantel 35 über, der die Stirnwand 33 berührungsfrei durchsetzt und in den Ringraum 29 ragt. Innerhalb des Stößelmantels 35 ist ein schaftförmiger Stößelkern 36 angeordnet, der mit einem in der Weite reduzierten Vorsprung 37 über das innere Ende des Stößelmantels 35 vorsteht und mit seinem anderen Ende einen Bestandteil einer Dralleinrichtung für die Auslaßöffnung 14 bilden kann. Der Auslaßkanal 15 durchsetzt den Vorsprung 37 in seiner Endfläche, die zur Bildung einer Spitze 38 abgeschrägt ist.

Im Übergangsbereich zum Vorsprung 37 bildet der Stößelkern 36 mit dem Stößelmantel 35 eine ringförmige Schulter 39, die abgedichtet an der zugehörigen, ebenen Stirnfläche des jeweiligen Pumpkolbens 9 anliegen kann. Der Vorsprung 37 ist eng an die Durchgangsöffnung 16 angepaßt und kann wie diese zylindrisch oder konisch sein. Das Ende des Vorsprunges 37 liegt in Ausgangslage in geringem Abstand von der inneren Stirnseite des Gehäusekörpers 2.

Die beiden Gehäusekörper 2, 3 sind durch ein durch die Folgeschaltung 10 gebildetes Gesperre 40 nur in einer Richtung gegeneinander verdrehbar. Sie bilden mit den äußeren Stirnflächen ihrer Stirnwände 26, 33 jeweils eine Druck-Handhabe 41, 42 und mit ihrem Außenumfang jeweils eine Dreh-Handhabe 43 bzw. 44; in Pumphubendlage liegt der Gehäusekörper 2 und damit dessen Handhabe 43 im wesentlichen vollständig innerhalb des Gehäusekörpers 3. Am Außenumfang des Mantels 19 sind zungenförmig frei und schräg entgegen der Drehrichtung des Gehäusekörpers 2 vorsehende Rastglieder 45 verteilt vorgesehen, denen am Innenumfang des Mantels 28 eine über dessen gesamte Länge durchgehende Zahnung 46 mit einer Teilung zugeordnet ist, die der Teilung der Halterungen 23 entspricht. Die Zahnung 46 bildet nach innen vorstehende Zähne bzw. Rastglieder 47, die jeweils an einer Flanke von einer flach ansteigenden Gleitfläche und an der anderen Flanke von einer annähernd radial zur Achse 21 liegenden Sperrschulter 48 begrenzt sind.

Die einteilig mit dem Mantel 19 ausgebildeten Rastglieder 45 sind nur an einem kurzen, an das innere Ende anschließenden Abschnitt des Gehäusekörpers 2 vorgesehen, so daß sie in Ausgangslage vollständig innerhalb des Gehäusekörpers 3 liegen.

In einer Raststellung der Folgeschaltung 10 bzw. des Gesperres 40 liegt der Vorsprung 37 achsgleich zu einer Pumpe 6. Werden nunmehr die Gehäusekörper 2, 3 entgegen der Kraft einer Rückstellfeder 50 zusammengedrückt, so tritt der Vorsprung 37 in den Pumpkolben 9 ein, durchsticht den Verschluß 17 und nimmt dann über die Schulter 39 den Pumpkolben 9 bis zur Anlage am Boden des Behälters 13 mit, da der Vorsprung 37 im wesentlichen nicht über das innere Ende des Pumpkolbens 9 vorsteht. Während dieser Pumphubbewegung wird der Inhalt der Pumpenkammer 12 durch den Auslaßkanal 15 und die Auslaßdüse vollständig ausgetragen.

Durch die beschriebene Ausbildung ist der Stößel 11 geringfügig federnd auslenkbar, so daß er sich beim Kuppeln von selbst genau gegenüber dem Pumpkolben 9 ausrichten kann. Werden danach die Handhaben 41, 42 wieder freigegeben, so wird der Vorsprung 37 wieder aus dem Behälter 13 herausgezogen. Durch die sägezahnförmige Ausbildung der Kolbenlippen ist hierbei die Reibung gegenüber dem Behälter 13 so groß, daß sich der Stößel 11 bereits zu Beginn des Rückhubes wieder vom Pumpkolben 9 löst und dieser in seiner Endlage stehen bleibt.

In Ausgangslage können dann die beiden Gehäusekörper 2, 3 um einen Schaltschritt der Folgeschaltung 10 gegeneinander verdreht werden, wonach der Stößel 11 fluchtend zu einer weiteren Pumpe 6 steht. Während der Hubbewegungen bleibt die Drehsperre aufrechterhalten, weil die Rastglieder 45 an den Sperrschultern 48 gleiten.

Durch die magazinartige Ausbildung des Gehäuseteiles 2 können auch Pumpeneinheiten mit unterschiedlichen Wirkstoffen oder Wirkstoffkonzentrationen aufgenommen werden, und außerdem läßt sich das Magazin nach Trennen der Gehäuseteile 2, 3 auch wieder mit frischgefüllten Pumpeneinheiten nachrüsten.

Die Rückstellfeder 50 liegt seitlich benachbart und achsparallel zu den Pumpen 6 in der Achse 21. Sie ist im Mantel 27 zentriert und stützt sich an den Innenseiten der Stirnwände 26, 33 ab. Dadurch kann die außerhalb der Pumpenachsen liegende Rückstellfeder 50 die Pumpen 6 an einem oder beiden Enden überragen. Die Austragvorrichtung 1 läßt sich sehr einfach herstellen, montieren sowie handhaben und weist eine sehr kompakte Ausbildung auf. Der Auslaßkanal 15 ist im dargestellten Ausführungsbeispiel ventilfrei bzw. durchgehend offen ausgebildet, kann aber auch mit einem z.B. druckabhängig arbeitenden Auslaßventil versehen sein.

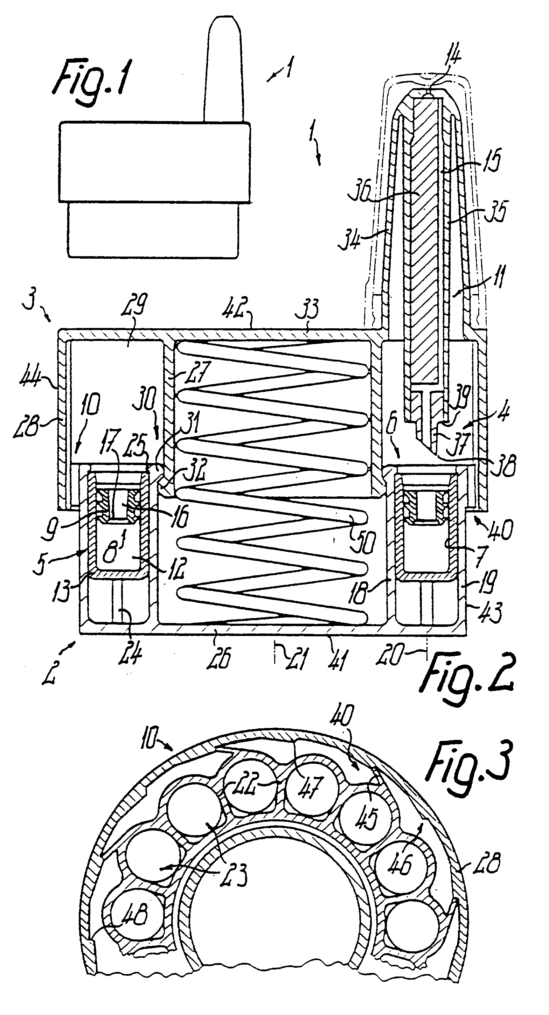

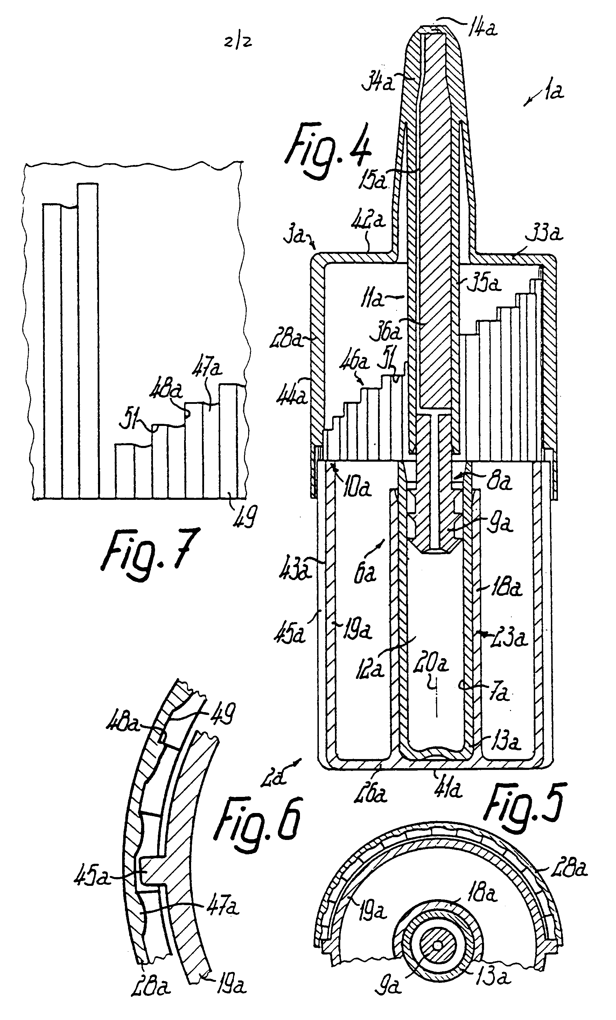

Die Austragvorrichtung nach den Figuren 1 bis 3 kann auch mit Merkmalen der Austragvorrichtung nach den Figuren 4 bis 7 z.B. so kombiniert werden, daß der jeweilige Pumpkolben 9 über zwei oder mehr aufeinaderfolgende Teilstrecken seines Gesamthubes jeweils anschlagbegrenzt betätigt werden kann, wonach durch Betätigen einer weiteren Folgeschaltung der nächste Teilhub ausgeführt werden kann. In den Figuren 4 bis 7 sind für einander entsprechende Teile die gleichen Bezugszeichen wie in den Figuren 1 bis 3, jedoch mit dem Index "a" verwendet, wobei die Beschreibungen sinngemäß für beide Ausführungsformen gelten.

Bei der Austragvorrichtung 1a ist nur eine einzige Pumpe 6a mit Pumpkolben 8a und Pumpenzylinder 7a vorgesehen, die in der Achse 20a liegen. Der Behälter 13a weist eine gegenüber seinem Durchmesser vielfach größere Länge auf, so daß die Pumpenkammer 12a durch schrittweises, jedoch rückstellungsfreies Einfahren des Pumpkolbens 9a entleert werden kann. Zu diesem Zweck weist die Folgeschaltung 10a am Innenumfang des Mantels 28a eine Vielzahl gleichmäßig über den Umfang verteilter Nuten 49 in zwei gleichen, jeweils über den halben Umfang sich erstreckenden Gruppen auf. In jeweils zwei gleichlange, einander diametral gegenüberüberliegende Nuten 49 greifen zwei Rastglieder 45a bzw. Längsstege ein, die am Außenumfang des Mantels 19a vorgesehen sind. Benachbarte Nuten 49 nehmen stufenweise in der Länge annähernd bis zur Stirnwand 33a zu und gehen von der offenen Stirnseite des Gehäusekörpers 3a aus.

Zwischen benachbarten Nuten 49 ist jeweils ein z.B. wulstförmig vorstehendes Rastglied 47a vorgesehen, das im wesentlichen über die gesamte Länge der kürzeren Nut reicht und dessen der Achse 20a zugekehrte Längskante konvex gekrümmt ist oder nach Art einer ansteigenden Zahnflanke ausgebildet sein kann, so daß es die jeweils längere Nut 49 seitlich mit einer Sperrschulter 48a begrenzt bzw. die zugehörige Flanke dieser Nut 49 durch die Sperrschulter gebildet ist. Der die benachbarte kürzere Nut überragende Endabschnitt dieser Nutflanke bildet ebenfalls diese Sperrschulter 48a. Die Endfläche jeder Nut 49 bildet einen Anschlag 51 für das Ende des zugehörigen Rastgliedes 45a, das in der Ebene der offenen Stirnseite des Gehäuseteiles 2a liegen kann.

Werden die beiden Gehäusekörper 2a, 3a durch Zusammendrücken betätigt, so schlagen die Enden der Rastglieder 45a an den Anschlägen 51 der zugehörigen Nuten 49 an. Danach können die beiden Gehäusekörper 2a, 3a nur in einer Richtung gegeneinander verdreht werden, nämlich so, daß die Rastglieder 45a nach federndem Überspringen der Rastglieder 47a in den Bereich der nächst längeren Nuten 49 gelangen. Nunmehr kann eine weitere Betätigung über einen entsprechenden Teilhub erfolgen, der sich aus der Längendifferenz zwischen den beiden benachbarten Nuten 49 ergibt.

Der Pumpkolben 9a ist in diesem Fall einteilig mit dem Stößelkern 36 ausgebildet und steht über die offene Stirnseite des Gehäusekörpers 3a vor. Der Behälter 13a reicht annähernd von der Stirnwand 26a bis zur offenen Stirnseite des Gehäusekörpers 2a. Von der Stirnwand 26a steht der Mantel 18a frei in den Gehäusekörper 2a vor und umgibt den Behälter 13a als Halterung 23a am Außenumfang eng. Der Gehäusekörper 2a ist länger als der Gehäusekörper 3a, so daß die Handhabe 43a auch in Endstellung noch über den Gehäusekörper 3a zugänglich vorsteht.

## Patentansprüche

1. Austragvorrichtung für Medien mit mindestens einer Austrageinheit (6, 7) zum Austrag wenigstens einer Austragcharge durch eine manuelle Hubbewegung oder dgl., wobei die Austrageinheit (6, 6a) eine Halterung (23, 23a) zur Lagesicherung eines Medien-Behälters (13, 13a) sowie zwei erste und zweite, gegeneinander bewegbare Körper (2, 3) aufweist, von denen der erste Körper (3) mit einem Medienauslaß (14) versehen ist, dadurch gekennzeichnet, daß mindestens eine Halterung (23, 23a) für wenigstens einen Medien-Behälter (13, 13a) an dem zweiten Körper (2, 2a) der Austrageinheit (6, 6a) vorgesehen ist.

2. Austragvorrichtung nach Anspruch 1, dadurch gekennzeichnet, daß sie für pumpbare Medien als Austrageinheit mindestens eine zur Durchführung eines Austraghubes durch die manuelle Hubbewegung anzutreibende Pumpe (6, 6a) für den Austrag aufeinanderfolgender Austragchargen durch jeweils einen von aufeinanderfolgenden Austragzyklen aufweist und insbesondere zur Freigabe einer Sperrung des jeweils folgenden Austragzyklus eine manuell zu betätigende Folgeschaltung (10, 10a) vorgesehen ist, wobei die Folgeschaltung vorzugsweise durch eine von der Hubbewegung unabhängige bzw. gesonderte Relativbewegung zweier Vorrichtungsteile gegeneinander zu betätigen ist.

3. Austragvorrichtung nach Anspruch 1 oder 2, dadurch gekennzeichnet, daß die Austrageinheit (6, 6a) mit der jeweiligen manuellen Hubbewegung von einer freigegebenen Ausgangsstellung über den den Austraghub einschließenden Austragzyklus bis zu einer weiteren Ausgangsstellung zu überführen und in dieser weiteren Ausgangsstellung für die folgende manuelle Hubbewegung gesperrt ist, daß zur Freigabe der manuellen Hubbewegung des jeweils folgenden Austragzyklus' eine Folgeschaltung (10, 10a) unabhängig vom Volumen der jeweiligen Austragcharge stufenweise weiterzuschalten ist und daß vorzugsweise zur manuellen Freigabe des jeweils folgenden Austragzyklus' und zur Durchführung der Hubbewegung eine gemeinsame Handhabe (42, 44) vorgesehen ist, die insbesondere zur Freigabe des jeweils folgenden Austragzyklus' in einer Richtung quer zur Hubbewegung zu betätigen ist.

4. Austragvorrichtung nach einem der vorhergehenden Ansprüche, dadurch gekennzeichnet, daß eine Zylinderanordnung (5) mit mindestens einem von Ansaugöffnungen freien Pumpenzylinder (7, 7a) vorgesehen ist, der mit einem Pumpkolben (9, 9a) eine nur mit einer Auslaßöffnung (14, 14a) leitungsverbindbare Pumpenkammer (12, 12a) bildet, wobei die Zylinderanordnung insbesondere im wesentlichen den gesamten Medienspeicher der Austragvorrichtung bildet, daß vorzugsweise mindestens ein Pumpenzylinder (7, 7a) an einem einem Pumpkolben (9, 9a) gegenüberliegenden Boden geschlossen bzw. als einteiliges Speichergefäß ausgebildet ist und daß ein Auslaßkanal (15, 15a) den Pumpkolben (9, 9a) durchsetzt, wobei insbesondere der jeweilige Pumpenzylinder (7, 7a) in eine eng an seinem Mantel anliegende Halterung (23, 23a) einer Handhabe (41, 43 bzw. 41a, 43a) eingesetzt ist.

5. Austragvorrichtung nach einem der vorhergehenen Ansprüche, dadurch gekennzeichnet, daß zwei kappenförmige, teleskopartig ineinandergreifende Handhaben (41, 42 bzw. 41a, 42a) vorgesehen sind, die insbesondere durch die gegeneinander bewegbaren Körper (2, 3) gebildet und/oder um Schalt-Schritte einer Folgeschaltung (10, 10a) gegeneinander verdrehbar sind sowie vorzugsweise zwischen ihren einander zugekehrten Mantelflächen Rastglieder (45, 47 bzw. 45a, 47a) aufweisen.

6. Austragvorrichtung nach einem der vorhergehenen Ansprüche, dadurch gekennzeichnet, daß mindestens ein Behälter (13) exzentrisch zu einer Mittelachse (21) einer Folgeschaltung (10) liegt, insbesondere ein zu einer Drehachse achsgleicher Kranz mit einer Mehrzahl von Behältern (13) wahlweise betätigbarer Pumpen (6) vorgesehen ist und daß vorzugsweise mehrere Behälter (13) wahlweise mit derselben Austragöffnung (14) verbindbar sind, die insbesondere mit der Folgeschaltung (10) gegenüber den Behältern (13) bewegbar gelagert ist und/oder am Ende eines einen Kolbenstößel (11) durchsetzenden Auslaßkanales (15) liegt.

7. Austragvorrichtung nach einem der vorhergehenen Ansprüche, dadurch gekennzeichnet, daß mindestens ein Pumpkolben (9) einer Austrageinheit (6) in einer Ausgangslage wenigstens von einem Teil eines Kolbenstößels (11) getrennt ist, insbesondere eine vom Kolbenstößel (11) gesonderte Baueinheit mit dem zugehörigen Behälter (13) bildet und daß vorzugsweise der Kolbenstößel (11) quer zu seiner Achse (20) gegenüber dem Pumpkolben (9) ausrichtbar und/oder etwa parallel zu dieser Achse (20) mit dem Pumpkolben (9) kuppelbar ist.

8. Austragvorrichtung nach einem der vorhergehenen Ansprüche, dadurch gekennzeichnet, daß mindestens eine Pumpenkammer (12, 12a) einer Austrageinheit (6, 6a) in einer Ausgangslage an einer Austragseite vollständig dicht Verschlossen und insbesondere ein mit dem Kolbenstößel (11) etwa am Beginn oder dgl. eines Pumphubes zu öffnender Verschluß (17) für eine Durchgangsöffnung (16) des Pumpkolbens (9) vorgesehen ist, daß der Verschluß (17) vorzugsweise durch Zerstörung zu öffnen, insbesondere eine Verschlugmembran, ist und insbesondere mit dem Pumpkolben (9) eine Baueinheit bildet bzw. vollständig innerhalb des Pumpkolbens (9) liegt und daß vorzugsweise der Kolbenstößel (11) zur Verbindung mit dem Pumpkolben (9) einen Vorsprung (37), insbesondere eine vom Auslaßkanal (15) durchsetzte Stechspitze für den Verschluß (17) aufweist, die insbesondere eng an die Durchgangsöffnung (16) des Pumpkolbens (9) angepaßt ist und ein Steckglied für die Verbindung mit dem Pumpkolben (9) bildet.

9. Austragvorrichung nach einem der vorhergehenen Ansprüche, dadurch gekennzeichnet, daß eine Folgeschaltung (10, 10a) für aufeinanderfolgende Austragchargen ein die Schaltbewegung nur in einer Richtung freigebendes und gegen die andere Richtung sperrendes Gesperre (40), insbesondere Rastglieder (45, 47) aufweist, die durch mindestens einen Rastnoken und wenigstens einen widerhakenartigen Rastvorsprung für dessen Eingriff gebildet sind und/oder daß eine Handhabe (41, 43) mit einem Ringabschnitt in einen Ringraum (29) einer anderen Handhabe (42, 44) eingreift und insbesondere die Handhaben im wesentlichen nur im Bereich einer z. B. radial inneren Umfangsbegrenzung des Ringraumes (29) aneinander geführt sowie im Bereich der anderen Umfangsbegrenzung Rastglieder (45, 47) und/oder ein Gesperre (40) vorgesehen sind, wobei vorzugsweise der Ringabschnitt einen Kranz von Halterungen (23) für Behälter (13) bildend in den Ringraum (29) der Kolbenstößel (11) frei vorsteht bzw. innerhalb der inneren Umfangsbegrenzung des Ringraumes (29) eine Rückstellfeder liegt, die an gegenüberliegenden Stirnwänden (26, 33) der Handhaben abgestützt ist.

10. Austragvorrichtung nach einem der vorhergehenen Ansprüche, dadurch gekennzeichnet, daß nur ein einziger Behälter (13a) vorgesehen ist und daß die Hubbewegung über abgestufte Anschläge (51) einer Folgeschaltung (10a) schrittweise in Teilhübe unterteilt ist, wobei vorzugsweise die Anschläge (51) im wesentlichen über den gesamten Innenumfang einer Handhabe (42a, 44a) verteilt sind.

11. Austragvorrichtung nach einem der vorhergehenden Ansprüche, dadurch gekennzeichnet, daß Mittel zur Erfassung der ausgebrachten Austragchargen vorgesehen sind, wobei Vorzugsweise eine Folgeschaltung (10, 10a) für aufeinanderfolgende Austragchargen zur Erfassung vorgesehen ist.

## Claims

1. Dispensing device for media with at least one dispensing unit (6, 7) for dispensing at least one dispensing batch through a manual stroke movement or the like, the dispensing unit (6, 6a) having a mounting support (23, 23a) for fixing the position of a medium container (13, 13a), as well as two first and second, mutually movable bodies (2, 3), whereof the first body (3) is provided with a medium outlet (14), characterized in that there is at least one mounting support (23, 23a) for at least one medium container (13, 13a) on the second body (2, 2a) of the dispensing unit (6, 6a).

2. Dispensing device according to claim 1, characterized in that for pumpable media as the dispensing unit it has at least one pump (6, 6a) drivable through the manual stroke movement for performing a dispensing stroke for the dispensing of successive dispensing batches through in each case one of successive dispensing cycles and in particular for the release of a locking means of the in each case following dispensing cycle is provided a manually operable sequence control (10, 10a), the sequence control preferably being operable by a relative movement with respect to one another of two device parts independent or separate from the stroke movement.

3. Dispensing device according to claim 1 or 2, characterized in that the dispensing unit (6, 6a) can be transferred with the manual stroke movement from a releasing starting position over the dispensing cycle including the dispensing stroke to a further starting position and in said further starting position is locked for the following manual stroke movement, that for the release of the manual stroke movement of the in each case following dispensing cycle a sequence control (10, 10a) can be stepwise advanced independently of the volume of the dispensing batch and that preferably for the manual release of the in each case following dispensing cycle and for performing the stroke movement a common handle (42, 44) is provided, which in particular for the release of the in each case following dispensing cycle is operable in a direction at right angles to the stroke movement.

4. Dispensing device according to one of the preceding claims, characterized in that a cylinder arrangement (5) with at least one pump cylinder (7, 7a) free from suction openings is provided, which forms with a plunger (9, 9a) a pump chamber (12, 12a) line-connectable only with one discharge opening (14, 14a), the cylinder arrangement forming substantially the entire medium reservoir of the dispensing device, that preferably at least one pump cylinder (7, 7a) is closed at a bottom facing a plunger (9, 9a) or constructed as a one-piece storage vessel and that a discharge channel (15, (15a) traverses the plunger (9, 9a) and in particular the pump cylinder (7, 7a) is inserted in a mounting support (23, 23a), closely engaging on its jacket, of a handle (41, 43 or 41a, 43a).

5. Dispensing device according to one of the preceding claims, characterized in that there are two cap-like, telescopically interengaging handles (41, 42 or 41a, 42a), which are in particular formed by the oppositely movable bodies (2, 3) and/or are rotatable against one another by steps of a sequence control (10, 10a) and preferably have detents (45, 47 or 45a, 47a) between their facing circumferential surfaces.

6. Dispensing device according to one of the preceding claims, characterized in that at least one container (13) is eccentric to a centre axis (21) of a sequence control (10) and in particular a ring equiaxial to a rotation axis with a plurality of containers (13) of selectively operable pumps (6) is provided and that preferably several containers (13) can be connected, as desired, with the same discharge opening (14), which is in particular movably mounted with the sequence control (10) with respect to the containers (13) and/or is located at the end of a discharge channel (15) traversing a push rod (11).

7. Dispensing device according to one of the preceding claims, characterized in that at least one plunger (9) of a dispensing unit (6) in one starting position is separated from at least part of a push rod (11) and in particular forms a unit separate from the push rod (11) with the associated container (13) and that preferably the push rod (11) can be oriented at right angles to its axis (20) with respect to the plunger (9) and/or can be coupled to the plunger (9) roughly parallel to said axis (20).

8. Dispensing device according to one of the preceding claims, characterized in that at least one pump chamber (12, 12a) of a dispensing unit (6, 6a) in one starting position is completely tightly closed on one dispensing side and in particular a seal (17) for a through opening (16) of the plunger (9) opening with the push rod (11) roughly at the start of a pump stroke is provided, that the seal (17) is preferably opened by destruction, especially a closing membrane and in particular forms a unit with the plunger (9) or is located entirely within the plunger (9) and that preferably for connection to the plunger (9) the push rod (11) has a projection (37), particularly a piercing tip for the seal (17) traversed by the discharge channel (15) and which is in particular closely adapted to the through opening (16) of the plunger (9) and forms a plugging member for connection to the plunger (9).

9. Dispensing device according to one of the preceding claims, characterized in that a sequence control (10, 10a) has for successive dispensing batches a locking mechanism (40), particularly detents (45, 47) releasing the switching movement in only one direction and locking against the other direction, formed by at least one locking member and at least one barb-like locking projection for the engagement thereof and/or that a handle (41, 43) engages with a ring portion in an annular space (29) of another handle (42, 44) and in particular the handles are only guided on one another in the vicinity of an e.g. radially inner circumferential boundary of the annular space (29) and in the vicinity of the other circumferential boundary detents (45, 47) and/or a locking mechanism (40) are provided and preferably the annular portion, forming a ring of mounting supports (23) for containers (13), projects freely into the annular space (29) of the push rod (11) or within the inner circumferential boundary of the annular space (29) is provided a return spring, which is supported on opposite end walls (26, 33) of the handles.

10. Dispensing device according to one of the preceding claims, characterized in that there is only one container (13a) and that the stroke movement is subdivided stepwise into partial strokes over stepped stop members (51) of a sequence control (10a) and preferably the stop members (51) are distributed over the entire inner circumference of a handle (42a, 44a).

11. Dispensing device according to one of the preceding claims, characterized in that there are means for determining the discharged dispensing batches and preferably a sequence control (10, 10a) is provided for establishing the successive dispensing batches.

## Revendications

1. Dispositif de distribution ou dispensation pour matières avec au moins une unité de distribution (6, 7) pour la distribution ou dispensation d'au moins une charge de distribution par un mouvement de course manuelle ou semblable, l'unité de distribution (6, 6a) présentant un support (23, 23a) pour l'assurage de la position d'un récipient de matières (13, 13a), ainsi que deux premiers et deuxièmes corps (2, 3), mobiles l'un contre l'autre, desquels le premier corps (3) est muni d'un échappement pour matières (14), caractérisé en ce qu'au moins un support (23, 23a) est prévu pour au moins un récipient de matières (13, 13a) au deuxième corps (2, 2a) de l'unité de distribution (6, 6a).

2. Dispositif de distribution selon la revendication 1, caractérisé en ce qu'il présente, pour matières pompables, comme unité de distribution, au moins une pompe (6, 6a), pour l'exécution d'une course de distribution, actionable par le mouvement de course manuelle, pour la distribution de charges de distribution consécutives, par respectivement un des cycles de distribution consécutifs et notamment il est prévu, pour la libération d'un blocage du respectif cycle de distribution suivant, un commutateur à séquence (10, 10a), actionnable manuellement, étant le commutateur à sequence à actionner de préférence par un mouvement relatif indépendant respectivement séparé du mouvement de course de deux parties du dispositif l'une contre l'autre.

3. Dispositif de distribution selon la revendication 1 ou 2, caractérisé en ce que l'unité de distribution (6, 6a), avec le respectif mouvement de course manuelle d'une position initiale libérée, à travers un cycle de distribution, incluyant la course de distribution, est à transporter jusqu'à une suivante position de départ et en cette suivante position de départ est bloquée pour le suivant mouvement de course manuelle et en ce que pour la libération du mouvement de course manuelle du respectif cycle de distribution suivant, un commutateur à sequence (10, 10a) est graduellement à commuter indépendamment du volume de la respective charge de distribution et en ce que, de préférence, pour la libération manuelle du respectif cycle de distribution suivant et pour l'exécution du mouvement de course, est prévu une manette (42, 44) commune, laquelle notamment, pour la libération du respectif cycle de distribution suivant, est à actionner dans une direction transversale au mouvement de course.

4. Dispositif de distribution selon une des revendications précédentes, caractérisé en ce que une disposition de cylindres (5) est prévue avec au moins un cylindre de pompe (7, 7a) libre d'orifices d'aspiration, lequel, avec un piston de pompe (9, 9a) forme une chambre de pompe (12, 12a), raccordable par conduite seulement avec un échappement pour matières (14, 14a), la disposition de cylindres formant notamment, essentiellement, l'accumulateur pour matières total du dispositif de distribution et en ce que, de préférence, au moins un cylindre de pompe (7, 7a) est formé d'une façon close, respectivement comme récipient d'accumulation d'une pièce, sur un fond opposé à un piston de pompe (9, 9a) et en ce que un canal d'echappement (15, 15a) traverse le piston de pompe (9, 9a), étant en particulier le respectif cylindre de pompe (7, 7a) inséré dans un support (23, 23a) d'une manette (41, 43 respectivement 41a, 43a) contigu étroitement à son manteau.

5. Dispositif de distribution selon une des revendications précédentes, caractérisé en ce que deux manettes (41, 42 respectivement 41a, 42a) sont prévues en forme de capuchon, qui se saisissent l'une dans l'autre d'une façon telescopique, lesquelles notamment sont formées par les corps (2, 3) mobiles l'un contre l'autre et/ou sont rotatifs par pas de commutation d'un commutateur à séquences (10, 10a) ainsi que de préférence ils présentent entre leur surfaces de manteau, tournées l'une vers l'autre, des éléments d'arrêts (45, 47 respectivement 45a, 47a).

6. Dispositif de distribution selon une des revendications précédentes, caractérisé en ce qu'au moins un récipient (13) est situé excentriquement par rapport à un axe central (21) d'un commutateur à séquences (10), est prévue en particulier une couronne, ayante le même axe qu'un axe de rotation, avec une multiplicité de récipients (13) de pompes (6) actionnables sélectivement et en ce que, de préférence, plusieurs récipients (13) sont reliables avec la même ouverture de distribution (14), laquelle est logé notamment mobilement par rapport aux récipients (13) avec le commutateur à séquences (10) et/ou est situé à la fin d'un canal d'echappement (15) traversant un poussoir de piston (11).

7. Dispositif de distribution selon une des revendications précédentes, caractérisé en ce qu'au moins un piston de pompe (9) d'une unité de distribution (6) dans une position de départ est séparé au moins d'une part d'un poussoir de piston (11), notamment forme une unité de construction séparée du poussoir de piston (11) avec le récipient (13) associé et en ce que de préférence le poussoir de piston (11) est orientable envers le piston de pompe (9) transversalement a son axe (20) et/ou est accouplable quasi parallèlement à cet axe (20) avec le piston de pompe (9).

8. Dispositif de distribution selon une des revendications précédentes, caractérisé en ce qu'au moins une chambre de pompe (12, 12a) est prévue d'une unité de distribution (6, 6a) dans une position de départ, fermée complètement hermétiquement d'un côté de distribution et notamment une fermeture (17), ouvrable avec le poussoir de piston (11) à peu près au commencement ou semblable d'une course de pompage, pour une ouverture de passage (16) du piston de pompe (9) et en ce que la fermeture (17), de préférence une membrane de fermeture, est de préférence ouvrable par destruction, et notamment forme une unité de construction avec le piston de pompe (9) respectivement est située complètement à l'intérieur du piston de pompe (9) et en ce que de préférence le poussoir de piston (11) présente, pour la connection avec le piston de pompe (9) une partie en saillie (37), notamment une pointe perforante traversée par un canal d'echappement (15) pour la fermeture (17), laquelle notamment est strictement adaptée à l'ouverture de passage (16) du piston de pompe (9) et forme un élément d'insertion pour la connection avec le piston de pompe (9).

9. Dispositif de distribution selon une des revendications précédentes, caractérisé en ce qu'un commutateur à séquence (10, 10a) pour charges de distribution consécutives présente un arrêt (40), libérant le mouvement de commande seulement dans une direction et bloquant dans l'autre direction, notamment des éléments d'arrêt (45, 47), qui sont formés au moins d'une came d'arrêt et au moins d'une partie en saillie d'arrêt à façon de barbe pour leur prise et/ou en ce qu'une manette (41, 43) avec une partie annulaire intervient dans un espace annulaire (29) d'une autre manette (42, 44) et notamment les manettes essentiellement sont guidées adjacentes seulement dans le champ d'une limitation circonférencielle, par exemple radiale intérieure, de l'espace annulaire (29) ainsi que sont prévus, dans le champ de l'autre limitation circonférencielle, des éléments d'arrêt (45, 47) et/ou un arrêt (40), la partie annulaire de préférence formant une couronne de supports (23) pour récipients (13), sort librement dans l'espace annulaire (29) des poussoirs de piston (11) respectivement est situé à l'intérieur de la limitation circonférencielle intérieure de l'espace annulaire (29) un ressort de rappel, lequel est soutenu aux parois frontales (26, 33) opposées des manettes.

10. Dispositif de distribution selon une des revendications précédentes, caractérisé en ce que seulement un récipient (13a) est prévu et en ce que le mouvement de course est divisé par des arrêts (51) gradués d'un commutateur à séquence (10a) en courses partielles à phases successives, étant les arrêts (51) de préférence essentiellement distribués sur la circonférence intérieure totale d'une manette (42a, 44a).

11. Dispositif de distribution selon une des revendications précédentes, caractérisé en ce que des moyens pour la détection de la distribution des charges de distribution sont prévus, étant de préférence prévu, pour la détection, un commutateur à séquence (10, 10a) pour charges de distribution consécutives.
